Europäisches Patentamt

European Patent Office

Office européen des brevets

(11) Veröffentlichungsnummer: **0 139 076 B1**

# EUROPÄISCHE PATENTSCHRIFT

(45) Veröffentlichungstag der Patentschrift: **01.07.92**

(51) Int. Cl.⁵: **C12N 15/66**, C12N 15/16

(21) Anmeldenummer: **84104063.7**

(22) Anmeldetag: **11.04.84**

(54) **Human-Parathyroidhormon (Human-PTH) produzierende Hybridvektor.**

(30) Priorität: **11.04.83 DE 3312928**

(43) Veröffentlichungstag der Anmeldung:
**02.05.85 Patentblatt 85/18**

(45) Bekanntmachung des Hinweises auf die
Patenterteilung:
**01.07.92 Patentblatt 92/27**

(84) Benannte Vertragsstaaten:
**AT BE CH DE FR GB IT LI SE**

(56) Entgegenhaltungen:
EP-A- 0 009 930
EP-A- 0 037 723
EP-A- 0 038 182
WO-A-84/01173

Int. Congr. Ser.- Excerpta Med. (1980), pp.
5-18

Proc. Natl. Acad. Sci. USA (1981), vol. 78, pp.
7365-7369

Experientia, Band 39, Nr. 6 Juni 1983, Seite
659, Birkhäuser Verlag, Basel, CH. W. Born et
al "Expression and processing of human
preproparathyroid hormone in Escherichia
coli"

(73) Patentinhaber: **Gesellschaft für Biotechnologische Forschung mbH (GBF)
Mascheroder Weg 1
W-3300 Braunschweig-Stöckheim(DE)**

(72) Erfinder: **Mayer, Hubert, Dr.
Grosser Zimmerhof 10
W-3340 Wolfenbüttel(DE)**

(74) Vertreter: **Boeters, Hans Dietrich, Dr. et al
Boeters & Bauer Bereiteranger 15
W-8000 München 90(DE)**

Rank Xerox (UK) Business Services

EP 0 139 076 B1

Chemical abstracts, Band 94, Nr. 9, 2 März 1981, Seite 477, Ref.Nr. 62752w; Columbus, Ohio, US. H.M. Kronenberg et al "Cloning bovine and human parathyroid hormone DNA in bacteria" & Endocrinol. Proc. Int. Congr. Endocrinol., &th 1980, 508-11

Chemical abstracts, Band 100, Nr. 3, 16. Januar 1984, Seite 148, Ref.Nr. 18646h, Columbus, Ohio, US, H. Mayer et al "Assignment of the human parathyroid hormone gene to chromosome 11" & Hum. Genet. 1983, 64(3), 283-5

Proc. Natl. Acad. Sci. USA, Band 80, April 1983, Seiten 2127-2131, T.J. Vasicek et al "Nucleotide sequence of the human parathyroid hormone gene"

**Beschreibung**

Human-Parathyroidhormon reguliert u.A. den Einbau und Ausbau von Calcium in Knochen. Die Wirkungen und Funktionen von Human-Parathyroidhormon und seinen Agonisten und Antagonisten werden von Dambacher, Praktische Osteologie, Thieme Verlag Stuttgart / New York 1982; Reeve et al., British Medical J. 1340 (1980) 1 - 11; und Potts et al., Advances in Protein Chemistry, 35 (1982) 323 - 395 beschrieben.

In Int. Congr. Ser.-Excerpta Med., (1980) 5-18 wird die Expression von Pre-Pro-bPTH und Pro-bPTH beschrieben. In Proc. Natl. Acad. Sci. USA, 78 (1981) 7365-7369 wird die DNA-Sequenz von hPre-Pro-PTH beschrieben. Diese Beschreibung reichte jedoch nicht aus, um biologisch aktives hPTH zu exprimieren; vergl. Mol. Biol. Radiochem. Essays Calciotropic Horm. Sattelite Symp., (1984) S 80287.

Aufgabe der Erfindung ist es, biologisches Material zur Verfügung zu stellen, mit dem Human-Parathyroidhormon (hPTH) technisch hergestellt werden kann.

Gemäß der vorliegenden Erfindung wird diese Aufgabe durch einen in prokaryotischen Zellen klonierbaren Hybridvektor gelöst, der Human-Parathyroidhormon produziert und durch folgende Merkmale gekennzeichnet ist:

(a) einen Promotor

(b) einen sich an den Promotor anschließenden DNA-Bereich von 0 bis 1000 oder von 0 bis 200 Basenpaaren

(c) eine ribosomale Bindungsstelle, die sich an den DNA-Bereich gemäß b oder sofern b fehlt, an den Promotor gemäß a anschließt;

(d) einen sich an die ribosomale Bindungsstelle anschließenden DNA-Bereich von 4 bis 15 Basenoaaren,

(e) ein sich an den DNA-Bereich gemäß (d) anschließendes Startcodon und

(f) die folgende Human-Parathormon hyroid kodierende DNA-Sequenz:

```
Ser  Val  Ser  Glu  Ile  Gln  Leu  Met  His  Asn  Leu  Gly  Lys  His
TCT  GTG  AGT  GAA  ATA  CAG  CTT  ATG  CAT  AAC  CTG  GGA  AAA  CAT
AGA  CAC  TCA  CTT  TAT  GTC  GAA  TAC  GTA  TTG  GAC  CCT  TTT  GTA


Leu  Asn  Ser  Met  Glu  Arg  Val  Glu  Trp  Leu  Arg  Lys  Lys  Leu
CTG  AAC  TCG  ATG  GAG  AGA  GTA  GAA  TGG  CTG  CGT  AAG  AAG  CTG
GAC  TTG  AGC  TAC  CTC  TCT  CAT  CTT  ACC  GAC  GCA  TTC  TTC  GAC


Gln  Asp  Val  His  Asn  Phe  Val  Ala  Leu  Gly  Ala  Pro  Leu  Ala
CAG  GAT  GTG  CAC  AAT  TTT  GTT  GCC  CTT  GGA  GCT  CCT  CTA  GCT
GTC  CTA  CAC  GTG  TTA  AAA  CAA  CGG  GAA  CCT  CGA  GGA  GAT  CGA


Pro  Arg  Asp  Ala  Gly  Ser  Gln  Arg  Pro  Arg  Lys  Lys  Glu  Asp
CCC  AGA  GAT  GCT  GGT  TCC  CAG  AGG  CCC  CGA  AAA  AAG  GAA  GAC
GGG  TCT  CTA  CGA  CCA  AGG  GTC  TCC  GGG  GCT  TTT  TTC  CTT  CTG


Asn  Val  Leu  Val  Glu  Ser  His  Glu  Lys  Ser  Leu  Gly  Glu  Ala
AAT  GTC  TTG  GTT  GAG  AGC  CAT  GAA  AAA  AGT  CTT  GGA  GAG  GCA
TTA  CAG  AAC  CAA  CTC  TCG  GTA  CTT  TTT  TCA  GAA  CCT  CTC  CGT


Asp  Lys  Ala  Asp  Val  Asn  Val  Leu  Thr  Lys  Ala  Lys  Ser  Gln
GAC  AAA  GCT  GAT  GTG  AAT  GTA  TTA  ACT  AAA  GCT  AAA  TCC  CAG  T
CTG  TTT  CGA  CTA  CAC  TTA  CAT  AAT  TGA  TTT  CGA  TTT  AGG  GTC  A
```

Als prokaryotische Zellen kommen alle Zellen in Betracht, in denen sich Hybridvektoren mit den angegebenen Merkmalen in technischem Maßstab unter Bildung von Human-Parathyroidhormon klonieren lassen. Einzelheiten zum Klonieren und zur Expression von Genen unter der Steuerung von E.-coli-Promotoren (beispielsweise in Streptomyces) lassen sich bei Chater, Nature, 299 (1982) 10 ff finden.

EP 0 139 076 B1

Insbesondere kommt Escherichia coli in Betracht. Beispiele für geeignete Promotoren für E. coli finden sich beispielsweise bei Sengbusch, P. von, Molekular- und Zellbiologie, Springer-Verlag, Heidelberg etc. 1979. Bei E. coli kann die ribosomale Bindungsstelle (AB) beispielsweise die folgende DNA-Sequenz aufweisen:

$$\text{AGGA oder GGAG}$$

$$\text{TCCT} \qquad \text{CCTC}$$

Ein Beispiel für ein bei E. coli verwendbares Startcodon hat die DNA-Sequenz
ATG
TAG

Gemäß einer speziellen Ausführungsform der Erfindung kann die spezifische DNA-Sequenz gemäß (f) durch eine DNA-Sequenz ersetzt sein, deren Einzelstränge mit den Einzelsträngen der spezifischen DNA-Sequenz hybridisiert werden können, wobei die ersetzende DNA-Sequenz gleichfalls Human-Parathyroidhormon exprimiert.

Gemäß einer weiteren speziellen Ausführungsform der Erfindung können bei der spezifischen DNA-Sequenz gemäß (f) ein oder mehrere Basentripletts durch Basentripletts ersetzt sein, die die gleiche Aminosäure kodieren.

Nachstehend wird die Herstellbarkeit des erfindungsgemäßen Hybridvektors an einem Beispiel und drei Schemata näher erläutert.

Beispiel

Aus frisch geschlachteten Schweinen wurden die Nebenschilddrüsen operiert. Aus diesem Drüsengewebe wurde mRNA isoliert. Die isolierte mRNA wurde als doppelstrangige komplementäre DNA (ds-cDNA) mit Hilfe eines Plasmids in E. coli kloniert. Aus den erhaltenen Hybridklonen wurde die Hybrid-Plasmid-DNAs isoliert und in ihrer Einzelstrangform an einen Träger fixiert. Aus den Nebenschilddrüsen isolierte mRNA wurde mit fixierter Einzelstrang-DNA hybridisiert und entfernt und in einem In-Vitro-Translationssystem in das Schweine-Prä-Pro-Parathyroidhormon oder Schweine-Parathyroidhormon übersetzt. Das Schweine-Prä-Pro-Parathyroidhormon oder Schweine-Parathyroidhormom wurde durch Antikörperfällung nachgewiesen. Mit Hilfe einer "Hybrid-Arrested-Translation" und DNA-Sequenzanalyse konnten die Klone aufgefunden werden, die Schweine-Parathyroidhormonsequenzen enthielten. Aus den ermittelten Hybridklonen wurde Hybridplasmid-DNA, die das Schweine-cDNA Parathyroidhormongen (Schema 1) umfaßte, radioaktiv markiert (nick-translatiert) und zum Screenen von Humangenbänken verwendet. Auf diese Weise ermitteltes Human-Parathyroidhormongen wurde durch Subklonieren in einem Plasmid angereichert. Ein auf diese Weise angereichertes Human-Parathyroidhormongen wurde sequenziert. Die Sequenz des für die Expression von Human-Parathyroidhormon relevanten Abschnittes ist dem Schema 2 zu entnehmen. Die ermittelte Sequenz stimmte mit der bekannten cDNA-Sequenz und mit der bekannten Aminosäuresequenz des Human-Parathyroidhormons überein.

Im folgenden wird Schema 3 erläutert. (1) In das weitere Verfahren wurde die zwischen zwei Eco RI-Schnittstellen liegende DNA-Sequenz eingesetzt, die die DNA-Sequenz des Schemas 2 umfaßt. Es wurde nun mit Hilfe der Restriktionsendonuklease Sau3A der Prä-Pro-Teil des PTH-Gens vom Gen des reifen 1-84-PTH getrennt (2). Durch Auffüllen von dATP und dGTP mit dem "Large fragment" der E.-coli-DNA-Polymerase-I (3) und anschließenden Abbau mit S1-Nuklease wurde der verbleibende Einzelstrangrest (GA) der klebrigen Enden (sticky ends) beseitigt, die vom Sau3A-Schnitt herrührten (GATC); dadurch wurde das Codon "TCT" für die Aminosäure 1 (Serin) des Human-PTH rekonstituiert (4). An dieses in der angegebenen Weise behandelte PTH-DNA-Fragment wurde ein DNA-Adaptor ligiert. Dadurch wurde dem Serin ein Methionin-Codon "ATG" direkt vorgeschaltet. Dieses Codon ist eines der wichtigen Signale für den Start der Synthese von PTH im Mikroorganismus (5). Das in der angegebenen Weise konstruierte PTH-Genfragment wurde in die ClaI-Spaltstelle von pBR322 subkloniert. Ausgewählt wurde ein Klon, dessen PTH-Gen vor der HindIII-Spaltstelle des pBR322 (6 bis 7) gegen den Uhrzeigersinn orientiert war. Dieser PTH-Klon wurde mit HindIII gespalten (7). Die "sticky ends" dieser Spaltstelle ließen Auffüllreaktionen mit vier verschiedenen Nukleotiden zu. In Kombination mit dem Abbau durch S1-Nuklease gelangte man zu Fragmenten mit aufgefüllten Enden (flush ends), deren Entfernung vom ATG-Codon 4 bis 10 Basenpaare betrug (8). Vor diese Variation an Fragmenten wurden zwei synthetische DNA-Adaptoren ligiert, und zwar /TCCCTAGGGA/ + /TCCCTAGGGA/(9). Diese Linker enthielten die Sequenz der ribosomalen Bindungsstelle,

4

ein weiteres wichtiges Signal für die Expression im Mikroorganismus. Dadurch entstand weiter eine BamHI-Spaltstelle (10). Diese war für die Klonierung hinter verschiedenen Promotoren (wie trp, tac,T5) beschriebener Vektoren geeignet (11).

Die transformierten E. coli-Zellen wurden im LB-Vollmedium in Gegenwart von Ampicillin (50 $\mu$g/ml) bis zur mittleren logarithmischen Phase angezogen und abzentrifugiert. Das Pellet wurde in einem Suspensionspuffer mit Guanidiniumhydrochlorid (3 M) suspendiert (ungefähr $10^{10}$ Zellen/ml); danach wurde mit Ultraschall (Sonifier) bis zu einer optischen Dichte ($OD_{650}$) aufgeschlossen, die etwa 1/3 der optischen Dichte zu Beginn entsprach. Dieser Zellaufschluß wurde abzentrifugiert. Der Überstand enthielt PTH. Das Protein wurde mit 5-prozentigem TCA gefällt und in 0,02 n Salzsäure gelöst. TCA-Reste wurden mit Äther ausgewaschen. Das aus Rohextrakt und nach Extraktion gewonnene PTH war immunologisch gegen Antikörper (spezifisch zu PTH-Fragmenten AS1-34, AS28-48 und AS48-68) wirksam (RIA) und war im Adenylcyclase-Test biologisch aktiv.

Das Human-Parathyroidhormongen wurde mit einem Vektor verknüpft (pBR322/SV40-Derivat) und mit Hilfe einer Calciumphosphat-Fällung in Affennierenzellen transformiert. Aus $10^9$ Zellen wurde PTH durch Extraktion gewonnen; es war im RIA positiv gegen Antikörper (spezifisch zu den PTH-Fragmenten AS28-48 und AS44-68).

Außerdem wurde das Human-Parathyroidhormongen (lambda-Humanhybrid-DNA) mit dem Thymidinkinasegen von Herpes simplex durch Co-Transformation in T3-Zellen transformiert; aus den Tk[+]-Klonen wurde durch DNA-Hybridisieren die Integration des Human-PTH-Gens identifiziert.

## Schema 1

```
Asp Thr Val Lys Val Met Val Val Met Leu Ala Ile Cys Phe
GAC ACA GTT AAA GTA ATG GTT GTC ATG CTT GCA ATT TGT TTT
CTG TGT CAA TTT CAT TAC CAA CAG TAC GAA CGT TAA ACA AAA


Leu Ala Arg Ser Asp Gly Lys Pro Val Lys Lys Arg Ser Val
CTT GCA AGA TCA GAT GGG AAG CCT GTT AAG AAG AGA TCT GTG
GAA CGT TCT AGT CTA CCC TTC GGA CAA TTC TTC TCT AGA CAC


Ser Glu Ile Gln Leu Met His Asn Leu Gly Lys His Leu Ser
AGT GAA ATA CAG CTT ATG CAT AAC CTG GGC AAA CAC CTG AGC
TCA CTT TAT GTC GAA TAC GTA TTG GAC CCG TTT GTG GAC TCG


Ser Leu Glu Arg Val Glu Trp Leu Arg Lys Lys Leu Gln Asp
TCT CTG GAG AGA GTG GAA TGG CTG CGA AAG AAG CTG CAG GAT
AGA GAC CTC TCT CAC CTT ACC GAC GCT TTC TTC GAC GTC CTA


Val His Asn Phe Val Val Leu Gly Ala Ser Ile Val His Arg
GTG CAC AAC TTT GTT GTT CTC GGA GCT TCT ATA GTT CAC AGA
CAC GTG TTG AAA CAA CAA GAG CCT CGA AGA TAT CAA GTG TCT


Asp Gly Gly Ser Gln Arg Pro Pro Lys Lys Glu Asp Asn Val
GAT GGT GGT TCC CAG AGA CCC CCA AAA AAG GAA GAC AAT GTC
CTA CCA CCA AGG GTC TCT GGG GGT TTT TTC CTT CTG TTA CAG


Leu Val Glu Ser His Gln Lys Ser Leu Gly Glu Ala Asp Lys
CTA GTT GAG AGC CAT CAA AAA AGT CTC GGA GAA GCA GAT AAA
GAT CAA CTC TCG GTA GTT TTT TCA GAG CCT CTT CGT CTA TTT


Ala Ala Val Gly
GCT GCT GTG GGG
CGA CGA CAC CCC
```

**completed**

6

```
TGTCTTTAGTTTACTCAGCATCAGCTACTAACATACCTGAACGAAGATCTTGTTCTAAGA
ACAGAAATCAAATGAGTCGTAGTCGATGATTGTATGGACTTGCTTCTAGAACAAGATTCT

CATTGTAT
GTAACATA                        Intron II ca. 400 bp


                    Met Ile Pro Ala Lys Asp Met Ala Lys Val Met
         GTG AAG ATG ATA CCT GCA AAA GAC ATG GCT AAA GTT ATG
         CAC TTC TAC TAT GGA CGT TTT CTG TAC CGA TTT CAA TAC

Ile Val Met Leu Ala Ile Cys Phe Leu Thr Lys Ser Asp Gly Lys
ATT GTC ATG TTG GCA ATT TGT TTT CTT ACA AAA TCG GAT GGG AAA
TAA CAG TAC AAC CGT TAA ACA AAA GAA TGT TTT AGC CTA CCC TTT

Ser Val Lys
TCT GTT AAG
AGA CAA TTC


Intron I      Lys Arg Ser Val Ser Glu Ile Gln Leu Met His Asn
ca. 30 bp     AAG AGA TCT GTG AGT GAA ATA CAG CTT ATG CAT AAC
              TTC TCT AGA CAC TCA CTT TAT GTC GAA TAC GTA TTG

Leu Gly Lys His Leu Asn Ser Met Glu Arg Val Glu Trp Leu Arg
CTG GGA AAA CAT CTG AAC TCG ATG GAG AGA GTA GAA TGG CTG CGT
GAC CCT TTT GTA GAC TTG AGC TAC CTC TCT CAT CTT ACC GAC GCA

Lys Lys Leu Gln Asp Val His Asn Phe Val Ala Leu Gly Ala Pro
AAG AAG CTG CAG GAT GTG CAC AAT TTT GTT GCC CTT GGA GCT CCT
TTC TTC GAC GTC CTA CAC GTG TTA AAA CAA CGG GAA CCT CGA GGA

Leu Ala Pro Arg Asp Ala Gly Ser Gln Arg Pro Arg Lys Lys Glu
CTA GCT CCC AGA GAT GCT GGT TCC CAG AGG CCC CGA AAA AAG GAA
GAT CGA GGG TCT CTA CGA CCA AGG GTC TCC GGG GCT TTT TTC CTT

Asp Asn Val Leu Val Glu Ser His Glu Lys Ser Leu Gly Glu Ala
GAC AAT GTC TTG GTT GAG AGC CAT GAA AAA AGT CTT GGA GAG GCA
CTG TTA CAG AAC CAA CTC TCG GTA CTT TTT TCA GAA CCT CTC CGT

Asp Lys Ala Asp Val Asn Val Leu Thr Lys Ala Lys Ser Gln
GAC AAA GCT GAT GTG AAT GTA TTA ACT AAA GCT AAA TCC CAG TGA
CTG TTT CGA CTA CAC TTA CAT AAT TGA TTT CGA TTT AGG GTC ACT

AAA TGA AAA CAG ATA TTG TCA GAG TTC TGC TCT AGA CAG TGT AGG
TTT ACT TTT GTC TAT AAC AGT CTC AAG ACG AGA TCT GTC ACA TCC

GCA ACA ATA CAT GCT GCT AAT TCA AAG CTC TAT TAA GAT TTC CAA
CGT TGT TAT GTA CGA CGA TTA AGT TTC GAG ATA ATT CTA AAG GTT

GTG CCA ATA TTT CTG ATA TAA CAA ACT ACA TGT AAT CCA TCA CTA
CAC GGT TAT AAA GAC TAT ATT GTT TGA TGT ACA TTA GGT AGT GAT

GCC ATG ATA ACT GCA ATT TTA ATT GAT TAT TCT GAT TCC ACT TTT
CGG TAC TAT TGA CGT TAA AAT TAA CTA ATA AGA CTA AGG TGA AAA

ATT CAT TTG AGT TAT TTT AAT TAT CTT TTC TAT TGT TTA TTC TTT
TAA GTA AAC TCA ATA AAA TTA ATA GAA AAG ATA ACA AAT AAG AAA

TTA AAG TAT GTT ATT GCA TAA TTT ATA AAA GAA TAA AAT TCG ACT
AAT TTC ATA CAA TAA CGT ATT AAA TAT TTT CTT ATT TTA AGC TGA

TTT AAA CCT CTC TTC TAC CTT AAA ATG TAA AAC AAA AAT GTA ATG
AAA TTT GGA GAG AAG ATG GAA TTT TAC ATT TTG TTT TTA CAT TAC

ATC ATA AGT CTA AAT AAA TGA AGT ATT TCT CAC TCA AA
TAG TAT TCA GAT TTA TTT ACT TCA TAA AGA GTG AGT TT


     ----- Prä-Pro          ——— PTH
```

Schema 3:

Klonierungsschema für eine Human-PTH-Genexpression in E. coli

1) Lambda-Humanhybrid

DNA-Sequenz innerhalb dieses mit RI herausgeschnittenen Bereichs: (folgendes Blatt)

```
TGTCTTTAGTTTACTCAGCATCAGCTACTAACATACCTGAACGAAGATCTTGTTCTAAGA
ACAGAAATCAAATGAGTCGTAGTCGATGATTGTATGGACTTGCTTCTAGAACAAGATTCT

CATTGTAT
GTAACATA    ─────────────────    Intron II ca. 400 bp    ─────────


                Met Ile Pro Ala Lys Asp Met Ala Lys Val Met
          GTG AAG ATG ATA CCT GCA AAA GAC ATG GCT AAA GTT ATG
          CAC TTC TAC TAT GGA CGT TTT CTG TAC CGA TTT CAA TAC

Ile Val Met Leu Ala Ile Cys Phe Leu Thr Lys Ser Asp Gly Lys
ATT GTC ATG TTG GCA ATT TGT TTT CTT ACA AAA TCG GAT GGG AAA
TAA CAG TAC AAC CGT TAA ACA AAA GAA TGT TTT AGC CTA CCC TTT

Ser Val Lys
TCT GTT AAG
AGA CAA TTC


Intron I     Lys Arg Ser Val Ser Glu Ila Gln Leu Met His Asn
ca. 80 bp    AAG AGA TCT GTG AGT GAA ATA CAG CTT ATG CAT AAC
             TTC TCT AGA CAC TCA CTT TAT GTC GAA TAC GTA TTG

Leu Gly Lys His Leu Asn Ser Met Glu Arg Val Glu Trp Leu Arg
CTG GGA AAA CAT CTG AAC TCG ATG GAG AGA GTA GAA TGG CTG CGT
GAC CCT TTT GTA GAC TTG AGC TAC CTC TCT CAT CTT ACC GAC GCA

Lys Lys Leu Gln Asp Val His Asn Phe Val Ala Leu Gly Ala Pro
AAG AAG CTG CAG GAT GTG CAC AAT TTT GTT GCC CTT GGA GCT CCT
TTC TTC GAC GTC CTA CAC GTG TTA AAA CAA CGG GAA CCT CGA GGA

Leu Ala Pro Arg Asp Ala Gly Ser Gln Arg Pro Arg Lys Lys Glu
CTA GCT CCC AGA GAT GCT GGT TCC CAG AGG CCC CGA AAA AAG GAA
GAT CGA GGG TCT CTA CGA CCA AGG GTC TCC GGG GCT TTT TTC CTT

Asp Asn Val Leu Val Glu Ser His Glu Lys Ser Leu Gly Glu Ala
GAC AAT GTC TTG GTT GAG AGC CAT GAA AAA AGT CTT GGA GAG GCA
CTG TTA CAG AAC CAA CTC TCG GTA CTT TTT TCA GAA CCT CTC CGT

Asp Lys Ala Asp Val Asn Val Leu Thr Lys Ala Lys Ser Gln
GAC AAA GCT GAT GTG AAT GTA TTA ACT AAA GCT AAA TCC CAG TGA
CTG TTT CGA CTA CAC TTA CAT AAT TGA TTT CGA TTT AGG GTC ACT

AAA TGA AAA CAG ATA TTG TCA GAG TTC TGC TCT AGA CAG TGT AGG
TTT ACT TTT GTC TAT AAC AGT CTC AAG ACG AGA TCT GTC ACA TCC

GCA ACA ATA CAT GCT GCT AAT TCA AAG CTC TAT TAA GAT TTC AAA
CGT TGT TAT GTA CGA CGA TTA AGT TTC GAG ATA ATT CTA AAG TTT

GTG CCA ATA TTT CTG ATA TAA CAA ACT ACA TGT AAT CCA TCA CTA
CAC GGT TAT AAA GAC TAT ATT GTT TGA TGT ACA TTA GGT AGT GAT

GCC ATG ATA ACT GCA ATT TTA ATT GAT TAT TCT GAT TCC ACT TTT
CGG TAC TAT TGA CGT TAA AAT TAA CTA ATA AGA CTA AGG TGA AAA

ATT CAT TTG AGT TAT TTT AAT TAT CTT TTC TAT TGT TTA TTC TTT
TAA GTA AAC TCA ATA AAA TTA ATA GAA AAG ATA ACA AAT AAG AAA

TTA AAG TAT GTT ATT GCA TAA TTT ATA AAA GAA TAA AAT TCG ACT
AAT TTC ATA CAA TAA CGT ATT AAA TAT TTT CTT ATT TTA AGC TGA

TTT AAA CCT CTC TTC TAC CTT AAA ATG TAA AAC AAA AAT GTA ATG
AAA TTT GGA GAG AAG ATG GAA TTT TAC ATT TTG TTT TTA CAT TAC

ATC ATA AGT CTA AAT AAA TGA AGT ATT TCT CAC TCA AA
TAG TAT TCA GAT TTA TTT ACT TCA TAA AGA GTG AGT TT


     ────── Prä-Pro        ─────── PTH
```

2) erneuter Schnitt (Sau 3A)

9

Intron    Lys                              Arg  Ser  Val

G AGGAG A AG A                      G A T C T G T G ·····

C T C C T C T T C T C T A G             AC AC ·····

3) Auffüllen mit dG und dA

Ser  Val

G A T C T G T G ·····

AG AC AC ·····

4) Abbau mit S1-Nuklease

Ser  Val

T C T G T G ·····

AG AC AC ·····

5) Ligation mit DNA-Adaptor

Met  Ser  Val

C A T C G A T G | T C T G T G ·····

G T A G C T A C | AG AC AC ·····

6) erneuter Schnitt (ClaI)

Met  Ser  Val

C G A T G T C T G T G ·····

T A C A G A C A C ·····

7) Subklonieren in die ClaI-Spaltstelle von pBR322 und erneuter Schnitt (HindIII)

ClaI  Met  Ser  Val

A G C T T C A T C G A T G T C T G T G ·····

A G T A G C T A C A G A C A C ·····

8) verschiedene Auffüllreaktionen

Met

T C A T C G A T G ·······
A G T A G C T A C ·······

Met

T T C A T C G A T G ·····
A A G T A G C T A C ·······

Met

C T T C A T C G A T G ·······
G A A G T A G C T A C ·······

Met

G C T T C A T C G A T G ·······
C G A A G T A G C T A C ·······

Met

A G C T T C A T C G A T G ·······
T C G A A G T A G C T A C ·······

9) Ligation mit DNA-Adaptoren (2 identischen Linkern)

BamHI                                        Met   Ser   Val

T C C C T A G G G A T C C C T A G G G A G C T T C A T C G A T G T C T G T G·····
A G G G A T C C C T A G G G A T C C C T C G A A G T A G C T A C A G A C A C·····

10) erneuter Schnitt (BamHI)

RS = ribosomale Bindungsstelle

Stop   RS                    Met   Ser   Val

G A T C C C T A G G G A G C T T C A T C G A T G T C T G T G·····
G G A T C C C T C G A A G T A G C T A C A G A C A C·····

11) Insertion in ein Plasmid nach einem starken Promotor

Promotor        Terminator

PTH-gene

100 bp

**Patentansprüche**

**Patentansprüche für folgende Vertragsstaaten : BE, CH, DE, FR, GB, IT, LI, SE**

1.  In prokaryotischen Zellen klonierbarer und Human-Parathyroidhormon produzierender Hybridvektor, *gekennzeichnet* durch folgende Merkmale
    (a) einen Promotor,
    (b) einen sich an den Promotor anschließenden DNA-Bereich von 0 bis 1000 oder von 0 bis 200 Basenpaaren,
    (c) eine sich an den DNA-Bereich gemäß (b) anschließende ribosomale Bindungsstelle,
    (d) einen sich an die ribosomale Bindungsstelle anschließenden DNA-Bereich von 4 bis 15 Basenpaaren,
    (e) ein sich an den DNA-Bereich gemäß (d) anschließendes Startkodon und
    (f) die folgende Human-Parathyroidhormon kodierende DNA-Sequenz:


```
Ser  Val  Ser  Glu  Ile  Gln  Leu  Met  His  Asn  Leu  Gly  Lys  His
TCT  GTG  AGT  GAA  ATA  CAG  CTT  ATG  CAT  AAC  CTG  GGA  AAA  CAT
AGA  CAC  TCA  CTT  TAT  GTC  GAA  TAC  GTA  TTG  GAC  CCT  TTT  GTA


Leu  Asn  Ser  Met  Glu  Arg  Val  Glu  Trp  Leu  Arg  Lys  Lys  Leu
CTG  AAC  TCG  ATG  GAG  AGA  GTA  GAA  TGG  CTG  CGT  AAG  AAG  CTG
GAC  TTG  AGC  TAC  CTC  TCT  CAT  CTT  ACC  GAC  GCA  TTC  TTC  GAC


Gln  Asp  Val  His  Asn  Phe  Val  Ala  Leu  Gly  Ala  Pro  Leu  Ala
CAG  GAT  GTG  CAC  AAT  TTT  GTT  GCC  CTT  GGA  GCT  CCT  CTA  GCT
GTC  CTA  CAC  GTG  TTA  AAA  CAA  CGG  GAA  CCT  CGA  GGA  GAT  CGA


Pro  Arg  Asp  Ala  Gly  Ser  Gln  Arg  Pro  Arg  Lys  Lys  Glu  Asp
CCC  AGA  GAT  GCT  GGT  TCC  CAG  AGG  CCC  CGA  AAA  AAG  GAA  GAC
GGG  TCT  CTA  CGA  CCA  AGG  GTC  TCC  GGG  GCT  TTT  TTC  CTT  CTG


Asn  Val  Leu  Val  Glu  Ser  His  Glu  Lys  Ser  Leu  Gly  Glu  Ala
AAT  GTC  TTG  GTT  GAG  AGC  CAT  GAA  AAA  AGT  CTT  GGA  GAG  GCA
TTA  CAG  AAC  CAA  CTC  TCG  GTA  CTT  TTT  TCA  GAA  CCT  CTC  CGT


Asp  Lys  Ala  Asp  Val  Asn  Val  Leu  Thr  Lys  Ala  Lys  Ser  Gln
GAC  AAA  GCT  GAT  GTG  AAT  GTA  TTA  ACT  AAA  GCT  AAA  TCC  CAG  T
CTG  TTT  CGA  CTA  CAC  TTA  CAT  AAT  TGA  TTT  CGA  TTT  AGG  GTC  A
```

**2.** Hybridvektor nach Anspruch 1, dadurch *gekennzeichnet*, daß der Hybridvektor in E. coli klonierbar ist.

**3.** Hybridvektor nach Anspruch 1 oder 2, dadurch *gekennzeichnet,* daß die spezifische DNA-Sequenz gemäß Anspruch 1 (f) durch eine DNA-Sequenz ersetzt ist, deren Einzelstränge mit den Einzelsträngen der spezifischen DNA-Sequenz hybridisiert werden können, wobei die ersetzende DNA-Sequenz gewünschte Produkte exprimieren kann.

**4.** Hybridvektor nach Anspruch 1, 2 oder 3, dadurch *gekennzeichnet,* daß ein oder mehrere Basentripletts durch Synonyme ersetzt sind.

**Patentansprüche für folgenden Vertragsstaat : AT**

**1.** Verfahren zur Herstellung eines in prokaryotischen Zellen klonierbaren und Human-Parathyroidhormon produzierenden Hybridvektors, *dadurch gekennzeichnet*, daß man einen Vektor mit folgenden Merkmalen versieht:
(a) einen Promotor,
(b) einen sich an den Promotor anschließenden DNA-Bereich von 0 bis 1000 oder von 0 bis 200 Basenpaaren,
(c) eine sich an den DNA-Bereich gemäß (b) anschließende ribosomale Bindungsstelle,
(d) einen sich an die ribosomale Bindungsstelle anschließenden DNA-Bereich von 4 bis 15 Basenpaaren,
(e) ein sich an den DNA-Bereich gemäß (d) anschließendes Startkodon und
(f) die folgende Human-Parathyroidhormon kodierende DNA-Sequenz:

```
Ser  Val  Ser  Glu  Ile  Gln  Leu  Met  His  Asn  Leu  Gly  Lys  His
TCT  GTG  AGT  GAA  ATA  CAG  CTT  ATG  CAT  AAC  CTG  GGA  AAA  CAT
AGA  CAC  TCA  CTT  TAT  GTC  GAA  TAC  GTA  TTG  GAC  CCT  TTT  GTA


Leu  Asn  Ser  Met  Glu  Arg  Val  Glu  Trp  Leu  Arg  Lys  Lys  Leu
CTG  AAC  TCG  ATG  GAG  AGA  GTA  GAA  TGG  CTG  CGT  AAG  AAG  CTG
GAC  TTG  AGC  TAC  CTC  TCT  CAT  CTT  ACC  GAC  GCA  TTC  TTC  GAC


Gln  Asp  Val  His  Asn  Phe  Val  Ala  Leu  Gly  Ala  Pro  Leu  Ala
CAG  GAT  GTG  CAC  AAT  TTT  GTT  GCC  CTT  GGA  GCT  CCT  CTA  GCT
GTC  CTA  CAC  GTG  TTA  AAA  CAA  CGG  GAA  CCT  CGA  GGA  GAT  CGA


Pro  Arg  Asp  Ala  Gly  Ser  Gln  Arg  Pro  Arg  Lys  Lys  Glu  Asp
CCC  AGA  GAT  GCT  GGT  TCC  CAG  AGG  CCC  CGA  AAA  AAG  GAA  GAC
GGG  TCT  CTA  CGA  CCA  AGG  GTC  TCC  GGG  GCT  TTT  TTC  CTT  CTG


Asn  Val  Leu  Val  Glu  Ser  His  Glu  Lys  Ser  Leu  Gly  Glu  Ala
AAT  GTC  TTG  GTT  GAG  AGC  CAT  GAA  AAA  AGT  CTT  GGA  GAG  GCA
TTA  CAG  AAC  CAA  CTC  TCG  GTA  CTT  TTT  TCA  GAA  CCT  CTC  CGT


Asp  Lys  Ala  Asp  Val  Asn  Val  Leu  Thr  Lys  Ala  Lys  Ser  Gln
GAC  AAA  GCT  GAT  GTG  AAT  GTA  TTA  ACT  AAA  GCT  AAA  TCC  CAG  T
CTG  TTT  CGA  CTA  CAC  TTA  CAT  AAT  TGA  TTT  CGA  TTT  AGG  GTC  A
```

**2.** Verfahren nach Anspruch 1, dadurch *gekennzeichnet,* daß der Hybridvektor in E. coli klonierbar ist.

**3.** Verfahren nach Anspruch 1 oder 2, dadurch *gekennzeichnet,* daß die spezifische DNA-Sequenz gemäß Anspruch 1 (f) durch eine DNA-Sequenz ersetzt ist, deren Einzelstränge mit den Einzelsträngen der spezifischen DNA-Sequenz hybridisiert werden können, wobei die ersetzende DNA-Sequenz gewünschte Produkte exprimieren kann.

4. Verfahren nach Anspruch 1, 2 oder 3, dadurch **gekennzeichnet,** daß ein oder mehrere Basentripletts durch Synonyme ersetzt sind.

**Claims**
**Claims for the following Contracting States BE, CH, DE, FR, GB, IT, LI, SE**

1. Hybrid vector which can be cloned in prokaryotic cells and which produces human parathyroid hormone, characterized by the following features:
   (a) a promoter,
   (b) a DNA region, adjacent to the promoter, of from 0 to 1000 or from 0 to 200 base pairs,
   (c) a ribosome binding site, adjacent to the DNA region according to (b),
   (d) a DNA region, adjacent to the ribosome binding site, of from 4 to 15 base pairs,
   (e) a start codon, adjacent to the DNA region according to (d), and
   (f) the following DNA sequence encoding human parathyroid hormone:

```
Ser Val Ser Glu Ile Gln Leu Met His Asn Leu Gly Lys His
TCT GTG AGT GAA ATA CAG CTT ATG CAT AAC CTG GGA AAA CAT
AGA CAC TCA CTT TAT GTC GAA TAC GTA TTG GAC CCT TTT GTA


Leu Asn Ser Met Glu Arg Val Glu Trp Leu Arg Lys Lys Leu
CTG AAC TCG ATG GAG AGA GTA GAA TGG CTG CGT AAG AAG CTG
GAC TTG AGC TAC CTC TCT CAT CTT ACC GAC GCA TTC TTC GAC


Gln Asp Val His Asn Phe Val Ala Leu Gly Ala Pro Leu Ala
CAG GAT GTG CAC AAT TTT GTT GCC CTT GGA GCT CCT CTA GCT
GTC CTA CAC GTG TTA AAA CAA CGG GAA CCT CGA GGA GAT CGA


Pro Arg Asp Ala Gly Ser Gln Arg Pro Arg Lys Lys Glu Asp
CCC AGA GAT GCT GGT TCC CAG AGG CCC CGA AAA AAG GAA GAC
GGG TCT CTA CGA CCA AGG GTC TCC GGG GCT TTT TTC CTT CTG


Asn Val Leu Val Glu Ser His Glu Lys Ser Leu Gly Glu Ala
AAT GTC TTG GTT GAG AGC CAT GAA AAA AGT CTT GGA GAG GCA
TTA CAG AAC CAA CTC TCG GTA CTT TTT TCA GAA CCT CTC CGT


Asp Lys Ala Asp Val Asn Val Leu Thr Lys Ala Lys Ser Gln
GAC AAA GCT GAT GTG AAT GTA TTA ACT AAA GCT AAA TCC CAG T
CTG TTT CGA CTA CAC TTA CAT AAT TGA TTT CGA TTT AGG GTC A
```

2. Hybrid vector according to claim 1, characterised in that it can be cloned in E. coli.

3. Hybrid vector according to claim 1 or 2, characterised in that the specific DNA sequence according to claim 1(f) is replaced by a DNA sequence the single strands of which can be hybridised with the single strands of the specific DNA sequence, the replacement DNA sequence being able to express desired products.

**4.** Hybrid vector according to claim 1, 2 or 3, characterised in that one or more base triplets are replaced by synonyms.

**Claims for the following Contracting State : AT**

**1.** Process for the preparation of a hybrid vector which can be cloned in prokaryotic cells and which produces human parathyroid hormone, characterised in that a vector is provided with the following features:

(a) a promoter,
(b) a DNA region, adjacent to the promoter, of from 0 to 1000 or from 0 to 200 base pairs,
(c) a ribosome binding site, adjacent to the DNA region according to (b),
(d) a DNA region, adjacent to the ribosome binding site, of from 4 to 15 base pairs,
(e) a start codon, adjacent to the DNA region according to (d), and
(f) the following DNA sequence encoding human parathyroid hormone:

```
Ser Val Ser Glu Ile Gln Leu Met His Asn Leu Gly Lys His
TCT GTG AGT GAA ATA CAG CTT ATG CAT AAC CTG GGA AAA CAT
AGA CAC TCA CTT TAT GTC GAA TAC GTA TTG GAC CCT TTT GTA


Leu Asn Ser Met Glu Arg Val Glu Trp Leu Arg Lys Lys Leu
CTG AAC TCG ATG GAG AGA GTA GAA TGG CTG CGT AAG AAG CTG
GAC TTG AGC TAC CTC TCT CAT CTT ACC GAC GCA TTC TTC GAC


Gln Asp Val His Asn Phe Val Ala Leu Gly Ala Pro Leu Ala
CAG GAT GTG CAC AAT TTT GTT GCC CTT GGA GCT CCT CTA GCT
GTC CTA CAC GTG TTA AAA CAA CGG GAA CCT CGA GGA GAT CGA


Pro Arg Asp Ala Gly Ser Gln Arg Pro Arg Lys Lys Glu Asp
CCC AGA GAT GCT GGT TCC CAG AGG CCC CGA AAA AAG GAA GAC
GGG TCT CTA CGA CCA AGG GTC TCC GGG GCT TTT TTC CTT CTG


Asn Val Leu Val Glu Ser His Glu Lys Ser Leu Gly Glu Ala
AAT GTC TTG GTT GAG AGC CAT GAA AAA AGT CTT GGA GAG GCA
TTA CAG AAC CAA CTC TCG GTA CTT TTT TCA GAA CCT CTC CGT


Asp Lys Ala Asp Val Asn Val Leu Thr Lys Ala Lys Ser Gln
GAC AAA GCT GAT GTG AAT GTA TTA ACT AAA GCT AAA TCC CAG T
CTG TTT CGA CTA CAC TTA CAT AAT TGA TTT CGA TTT AGG GTC A
```

**2.** Process according to claim 1, characterised in that the hybrid vector can be cloned in E. coli.

**3.** Process according to claim 1 or 2, characterised in that the specific DNA sequence according to claim 1(f) is replaced by a DNA sequence the single strands of which can be hybridised with the single strands of the specific DNA sequence, the replacement DNA sequence being able to express desired products.

**4.** Process according to claim 1, 2 or 3, characterised in that one or more base triplets are replaced by synonyms.

**Revendications**

15

# EP 0 139 076 B1

**Revendications pour les Etats contractants suivants : BE, CH, DE, FR, GB, IT, LI, SE**

1. Vecteur hybride, pouvant être cloné dans des cellules procaryotes et produisant l'hormone parathyroïdienne humaine, caractérisé par les particularités suivantes :

   (a) un promoteur,
   (b) une portion d'ADN, de 0 à 1 000 ou de 0 à 200 paires de bases, se raccordant au promoteur,
   (c) un site d'attachement ribosomal se raccordant à la portion d'ADN conforme à (b),
   (d) une portion d'ADN, de 4 à 15 paires de bases, se raccordant au site d'attachement ribosomal,
   (e) un codon d'initiation se raccordant à la portion d'ADN conforme à (d) et
   (f) la séquence suivante d'ADN codant pour l'hormone parathyroïdienne humaine :

```
Ser Val Ser Glu Ile Gln Leu Met His Asn Leu Gly Lys His
TCT GTG AGT GAA ATA CAG CTT ATG CAT AAC CTG GGA AAA CAT
AGA CAC TCA CTT TAT GTC GAA TAC GTA TTG GAC CCT TTT GTA


Leu Asn Ser Met Glu Arg Val Glu Trp Leu Arg Lys Lys Leu
CTG AAC TCG ATG GAG AGA GTA GAA TGG CTG CGT AAG AAG CTG
GAC TTG AGC TAC CTC TCT CAT CTT ACC GAC GCA TTC TTC GAC


Gln Asp Val His Asn Phe Val Ala Leu Gly Ala Pro Leu Ala
CAG GAT GTG CAC AAT TTT GTT GCC CTT GGA GCT CCT CTA GCT
GTC CTA CAC GTG TTA AAA CAA CGG GAA CCT CGA GGA GAT CGA


Pro Arg Asp Ala Gly Ser Gln Arg Pro Arg Lys Lys Glu Asp
CCC AGA GAT GCT GGT TCC CAG AGG CCC CGA AAA AAG GAA GAC
GGG TCT CTA CGA CCA AGG GTC TCC GGG GCT TTT TTC CTT CTG


Asn Val Leu Val Glu Ser His Glu Lys Ser Leu Gly Glu Ala
AAT GTC TTG GTT GAG AGC CAT GAA AAA AGT CTT GGA GAG GCA
TTA CAG AAC CAA CTC TCG GTA CTT TTT TCA GAA CCT CTC CGT


Asp Lys Ala Asp Val Asn Val Leu Thr Lys Ala Lys Ser Gln
GAC AAA GCT GAT GTG AAT GTA TTA ACT AAA GCT AAA TCC CAG T
CTG TTT CGA CTA CAC TTA CAT AAT TGA TTT CGA TTT AGG GTC A
```

2. Vecteur hybride suivant la revendication 1, caractérisé en ce que le vecteur hybride peut être cloné dans E. coli.

3. Vecteur hybride suivant la revendication 1 ou 2, caractérisé en ce que la séquence spécifique d'ADN conforme à la revendication 1 (f) est remplacée par une séquence d'ADN dont les brins individuels peuvent faire l'objet d'une hybridation avec les brins individuels de la séquence spécifique d'ADN, la section d'ADN remplaçante pouvant exprimer les produits voulus.

4. Vecteur hybride suivant la revendication 1, 2 ou 3, caractérisé en ce qu'un ou plusieurs triplets de bases sont remplacés par des synonymes.

**Revendications pour l'Etat contractant suivant : AT**

1. Procédé de préparation d'un vecteur hybride, pouvant être cloné dans des cellules procaryotes et produisant l'hormone parathyroïdienne humaine, caractérisé en ce qu'on utilise un vecteur comportant

16

les particularités suivantes :

    (a) un promoteur,

    (b) une portion d'ADN, de 0 à 1 000 ou de 0 à 200 paires de bases, se raccordant au promoteur,

    (c) un site d'attachement ribosomal se raccordant à la portion d'ADN conforme à (b),

    (d) une portion d'ADN, de 4 à 15 paires de bases, se raccordant au site d'attachement ribosomal,

    (e) un codon d'initiation se raccordant à la portion d'ADN conforme à (d) et

    (f) la séquence suivante d'ADN codant pour l'hormone parathyroïdienne humaine :

```
Ser Val Ser Glu Ile Gln Leu Met His Asn Leu Gly Lys His
TCT GTG AGT GAA ATA CAG CTT ATG CAT AAC CTG GGA AAA CAT
AGA CAC TCA CTT TAT GTC GAA TAC GTA TTG GAC CCT TTT GTA


Leu Asn Ser Met Glu Arg Val Glu Trp Leu Arg Lys Lys Leu
CTG AAC TCG ATG GAG AGA GTA GAA TGG CTG CGT AAG AAG CTG
GAC TTG AGC TAC CTC TCT CAT CTT ACC GAC GCA TTC TTC GAC


Gln Asp Val His Asn Phe Val Ala Leu Gly Ala Pro Leu Ala
CAG GAT GTG CAC AAT TTT GTT GCC CTT GGA GCT CCT CTA GCT
GTC CTA CAC GTG TTA AAA CAA CGG GAA CCT CGA GGA GAT CGA


Pro Arg Asp Ala Gly Ser Gln Arg Pro Arg Lys Lys Glu Asp
CCC AGA GAT GCT GGT TCC CAG AGG CCC CGA AAA AAG GAA GAC
GGG TCT CTA CGA CCA AGG GTC TCC GGG GCT TTT TTC CTT CTG


Asn Val Leu Val Glu Ser His Glu Lys Ser Leu Gly Glu Ala
AAT GTC TTG GTT GAG AGC CAT GAA AAA AGT CTT GGA GAG GCA
TTA CAG AAC CAA CTC TCG GTA CTT TTT TCA GAA CCT CTC CGT


Asp Lys Ala Asp Val Asn Val Leu Thr Lys Ala Lys Ser Gln
GAC AAA GCT GAT GTG AAT GTA TTA ACT AAA GCT AAA TCC CAG T
CTG TTT CGA CTA CAC TTA CAT AAT TGA TTT CGA TTT AGG GTC A
```

**2.** Procédé suivant la revendication 1, caractérisé en ce que le vecteur hybride peut être cloné dans E. coli.

**3.** Procédé suivant la revendication 1 ou 2, caractérisé en ce que la séquence spécifique d'ADN conforme à la revendication 1 (f) est remplacée par une séquence d'ADN dont les brins individuels peuvent faire l'objet d'une hybridation avec les brins individuels de la séquence spécifique d'ADN, la section d'ADN remplaçante pouvant exprimer les produits voulus.

**4.** Procédé suivant la revendication 1, 2 ou 3, caractérisé en ce qu'un ou plusieurs triplets de bases sont remplacés par des synonymes.